# NOUVEAU FASCICULE DE BREVET EUROPEEN

(11) **EP 0 317 376 B2**
(45) Date de publication et mention de la décision concernant l'opposition: **03.04.1996**
(45) Mention de la délivrance du brevet: 13.01.1993
(21) Numéro de dépôt: 88402643.6
(22) Date de dépôt: 20.10.1988
(51) Int. Cl.: C07K 1/22, C07K 14/435

(54) **Préparation de concentré de facteur IX humain de haute pureté et d'autres protéines plasmatiques**
Herstellung von hoch-gereingtem menschlichen Faktor IX sowie anderem Plasmaproteinkonzentrat
Preparation of a concentrate of high-purity human factor IX and of other plasma proteins

(30) Priorité: 23.10.1987 FR 8714665; 25.05.1988 FR 8806923
(43) Date de publication de la demande: 24.05.1989
(73) Titulaire: CENTRE REGIONAL DE TRANSFUSION SANGUINE DE LILLE, F-59012 Lille (FR)
(72) Inventeur: BURNOUF Thierry, F - 59136 Wavrin (FR); MICHALSKI Catherine, F - 59800 Lille (FR)
(74) Mandataire: Lepeudry-Gautherat, Thérèse

(56) Documents cités:
- EP-A- 0 137 428
- EP-A- 0 229 026
- GB-A- 1 460 607
- GB-A- 2 080 312
- J. BIOCHEM, vol. 97, no. 5, 1985, pages 1347-1355; N. HASHIMOTO et al.: "A method for systematic purification from bovine plasma of six vitamin K-dependent coagulation factors: prothrombin, factor X, factor IX, protein S, protein C, and protein Z"
- PREPARATIVE BIOCHEMISTRY, vol. 11, no. 4, 1981, pages 397-412, Marcel Dekker, Inc.; S.P. BAJAJ et al.: "A simplified procedure for purification of human prothrombin factor IX and factor X"
- CHEMICAL ABSTRACTS, vol. 107, no. 23, 7 décembre 1987, page 259, résumé no. 213990n, Columbus, Ohio, US; T. BURNOUF et al.: "Biochemical and biological properties of an alphal-antitrypsin concentrate", & VOX SANG. 1987, 52(4), 291-7
- Suomela, Eur. J. Biochem. (1976) 71, 145-154
- Pepper et al., Thrombosis Research (1977) 11, 687-692
- Anderson et al., Thrombosis Research (1975) 7, 451-459

## Description

La présente invention concerne l'obtention d'un concentré de Facteur IX de haute pureté par des techniques de chromatographie d'échange d'ions et de chromatographie d'affinité, utilisant comme produit de départ une fraction de plasma humain contenant du Facteur IX; le procédé de la présente invention permet également d'obtenir des fractions enrichies en alpha-antitrypsine, en protéines C et S, en facteur VII ou proconvertine, en Facteur II ou prothrombine et en Facteur X ou facteur de Stuart. Le procédé permet également de récupérer l'albumine, l'antithrombine III et les immunoglobulines.

La mise à disposition de concentrés de Facteur VII, d'une part, et de Facteur IX, d'autre part, dépourvus d'autres protéines à activités coagulantes est très bénéfique pour les patients déficitaires gant en Facteur VII qu'en Facteur IX (hémophiles B) En effet, ces deux catégories de maladie sont actuellement traitées par l'utilisation de concentrés de protéines plasmatiques du complexe prothrombinique (aussi appelé PPSB) renfermant quatre protéines coagulantes, les Facteurs II, VII, IX et X. Soumis lors de leurs épisodes hémorragiques a un traitement par le PPSB tant les malades atteints de déficit en Facteur VII, que ceux déficitaires en Facteur IX, reçoivent des doses importantes d'autres facteurs de coagulation (Facteurs II et X) dont ils ont par ailleurs, avant traitement, des taux physiologiques normaux. Ce surcroît de protéines coagulantes peut induire des risques de thrombose. Ce danger est particulièrement net chez les déficients en Facteur VII, car cette protéine a, in vivo, une demi-durée de vie relativement brève (7 à 9 heures) qui conduit, pour rétablir des taux physiologiques normaux, a injecter des doses massives de PPSB et donc de Facteurs II, IX et X. Ce phénomène est d'autant plus marqué que le PPSB est généralement pauvre en Facteur VII (10 à 18 Ul/ml contre 30 à 45 Ul/ml de Facteur X, par exemple). Des traitements à base de plasma total ont été proposés, mais ceux-ci induisent des risques notoires de contamination virale puisque le plasma thérapeutique n'est généralement pas inactivé vis-à-vis des virus pathogènes, et nécessitent les mêmes injections massives et répétées que le PPSB, conduisant à des surcharges de protéines plasmatiques annexes.

S'il existe certaines publications démontrant l'intérêt de la chromatographie d'échanges ions (DEAE) et d'affinité (héparine-Sépharose) (Andersson et al., Thrombosis Research ; 7, 451-459, 1975), il y a peu de concentrés thérapeutiques spécifiques en Facteur VII et Facteur IX. Il existe actuellement un concentré de Facteur VIL mais ce dérivé renferme des antigènes des Facteurs IX, II et X, de la protéine C et des doses importantes d'héparine. Une méthode de préparation d'un concentré de Facteur IX, de haute pureté, a été publiée (Menaché et al., Blood, 64, 1220-1227, 1984) ; elle utilise deux étapes d'adsorption chromatographique, la première "en batch" en présence de DEAE-Sephadex, la deuxième en colonne de sulfate de dextran.

Cette technique consiste dans les faits à purifier d'abord le PPSB (DEAE-Sephadex) puis à isoler le Facteur IX grâce à son affinité pour le sulfate de dextran. Cette méthode n'offre cependant pas tous les avantages de la chromatographie industrielle en colonne, du fait de l'adsorption par lot ("en batch") (méthode peu automatisable, peu reproductible), et nuit à la récupération optimisée d'autres fractions plasmatiques potentiellement utiles. Enfin, elle ne favorise pas la récupération simultanée de la proconvertine.

Bajaj et al (Preparative Biochemistry 11,4; 1981; 397-412) décrivent également un procédé de purification dont les premières étapes sont réalisées sur citrate de baryum et sulfate d'ammonium mais dont l'étape spécifique, sur héparine-agarose est réalisée en présence de benzamidine. L'addition de benzamidine, qui facilite la purification en préservant l'activité du Facteur IX (en inhibant les protéases), est incompatible avec un usage thérapeutique du produit résultant.

Le brevet GB 1 460 607 décrit plusieurs procédés de purification de facteurs sanguins, dont le Facteur IX, par chromatographies, à partir de la fraction de Cohn I ou III, c'est-à-dire une pâte résultant d'une précipitation à l'éthanol. Après purification sur DEAE-Sephadex et heparinesepharose, on obtient une fraction contenant surtout du Facteur IX activé, sauf quand on protège la molécule de Facteur IX par l'addition de benzamidine.

La Demanderessc a maintenant découvert qu'un procédé, faisant appel à des techniques chromatographiques classiques, pouvait être mis en oeuvre sur toute fraction de plasma humain contenant du Facteur IX pour donner, après traitement approprié de celle-ci, un Facteur IX de haute pureté, c'est-à-dire présentant une activité spécifique supérieure à 120 Ul/mg et largement dépourvu des Facteurs II, VII et X.

L'invention concerne donc un procédé de préparation de Facteur IX de haute pureté caractérisé en ce qu'on soumet une fraction de plasma humain a une étape de prépurification pour éliminer des contaminants lipidiques et protéiques puis on soumet cette fraction prépurifiée à un traitement par voie chromatographique, associant notamment une chromatographie d'échange d'anions et une chromatographie d'affinité.

Comme indiqué ci-dessus le procédé de l'invention s'applique à toute fraction plasmatique contenant du Facteur IX et en particulier aux fractions qui contiennent également es Facteurs II, X et VII de même que l'alpha-inter-inhibiteur de trypsine, la protéine S et la protéine C, qui peuvent alors être également séparés par ledit procédé.

Le traitement de prépurification est destiné a l' obtention d'une fraction dans laquelle l'activité spécifique du Facteur IX est supérieure ou égale à 0,5 Ul/mg. En général une activité spécifique de 1 Ul/mg environ permet un déroulement satisfaisant du procédé et l'obtention d'un Facteur IX ayant la pureté recherchée.

Le filtrat de cette colonne de prépurification contient l'albumine, les immunoglobulines, l'antithrombine III et l'alpha-antitrypsine, ces différentes substances pouvant être purifiées et concentrées ultérieurement.

Les étapes ultérieures de chromatographie sont réalisées en utilisant une chromatographie d'échanges d'ions sur DEAE Sepharose, la chromatographie pouvant être effectuée en présence d'héparine, suivie d'une chromatographie d'affinité, sur héparine-Sépharose.

On peut bien entendu prévoir à un stade quelconque du procédé un traitement d'inactivation virale par une technique bien connue de l'homme de l'art.

Il s'est toutefois avéré que l'on obtenait des résultats particulièrement satisfaisants en soumettant la fraction de plasma prépurifiée à un traitement au solvantdétergent, tel que décrit par exemple dans la demande de brevet européen n° 0 131 740.

Le procédé selon l'invention comprend une chromatographie sur résine échangeuse d'anions, sur un gel de DEAE-Sépharose; par élution avec un tampon de force ionique croissante, on récupère trois fractions enrichies respectivement en proconvertine (Facteur VII), en protéines C et S et en alpha-inter-inhibiteur de trypsine, et enfin en Facteurs IX, II et X.

La fraction riche en proconvertine est désorbée par un tampon renfermant une concentration de NaCl de 0,18 à 0,20 M et préférentiellement 0,19 M et à pH légèrement acide. Dès sa réception, il est souhaitable d'y adjoindre de l'antithrombine III humaine, ceci à une dose souhaitable de 1 Ul/ml (concentration finale). La fraction est ensuite concentrée et, à ce stade, le taux d'héparine est ajusté entre 5 et 10 Ul/ml, avantageusement à 5 Ul/ml.

Ce procédé permet d'obtenir, avec un rendement de l'ordre de 45 %, une proconvertine à activité spécifique de 0,7 Ul/mg de protéine au moins.

La récupération des autres fractions plasmatiques adsorbées sur la résine échangeuse d'anions s'opère par augmentation graduelle de la force ionique.

C'est ainsi que s'élue préférentiellement une fraction contenant la protéine C et la protéine S et dépourvue des autres facteurs de coagulation,II, VII, IX et X, en augmentant la concentration saline du tampon chromatographique. La concentration en sel, préférentiellement le chlorure de sodium, doit être comprise entre 0,26 et 0,30 M et est avantageusement ajustée à 0,28 M et à pH légèrement acide.

La fraction riche en Facteurs IX, II et X est éluée par nouvelle augmentation de la concentration saline du tampon chromatographique, celle-ci étant portée entre 0,34 et 0,38 M de chlorure de sodium, et préférentiellement 0,36 M, le pH étant ajusté à 8,0.

Le procédé selon l'invention comprend, après cette chromatographie sur résine échangeuse d'anions, une nouvelle purification mettant en oeuvre une chromatographie d'affinité permettant la séparation en particulier des Facteurs II et X. Cette chromatographie d'affinité est réalisée sur héparine-sépharose, en tampon citrate.

Cette chromatographie permet de séparer un filtrat contenant le Facteur II qui peut eventuellement être récupéré pour fabriquer de la thrombine.

Par augmentation de la concentration saline dans le tampon citrate et, plus précisément, en ajustant la quantité de chlorure de sodium dans la gamme 0,23 - 0,27 M et plus favorablement 0,25 M, on élue une fraction riche en Facteur X et pouvant contenir des inhibiteurs des protéines C et S et l'α-inter-inhibiteur de trypsine.

Enfin, en portant la concentration en NaCl à une dose de l'ordre de 0,43 - 0,47 M , ou plus et préférentiellement à 0,45 M, on élue une fraction riche en Facteur IX. Celle-ci est ensuite concentrée par ultrafiltration et lyophilisée.

La mise en oeuvre de ce procédé permet, en partant d'un surnageant de cryoprécipité renfermant du Facteur IX en quantité correspondant à une activité spécifique de 0,014 Ul/mg, d'obtenir un Facteur IX de haute pureté à environ 120 Ul/mg de protéine.

Pour favoriser la stabilité du Facteur IX on peut prévoir un ou plusieurs stabilisants qui le protègent d'une dégradation et évitent la perte de l'activité coagulante. On ajoute de préférence ces stabilisants au tampon d'élution.

Parmi divers produits évalués, l'arginine s'est révélée particulièrement efficace pour stabiliser le Facteur IX. On l'utilise de préférence à une dose minimale de 1 g/l, par exemple comprise entre 1 et 5 g/l.

Par ailleurs, il peut être également judicieux de protéger le Facteur IX, notamment relativement à sa lyophilisation, en ajoutant un autre stabilisant sitôt son élution de la colonne chromatographique. Dans ce cas on utilise de préférence la lysine, à une concentration d'au moins 0,5 g/l, par exemple comprise entre 0,5 et 2 g/l.

Le produit ainsi obtenu peut être avantageusement utilisé en thérapeutique, notamment pour le traitement de l'hémophilie B. Par rapport aux produits équivalents déjà utilisés, il présente l'avantage d'être exempt des autres facteurs de la coagulation, des protéines plasmatiques et des contaminants lipidiques.

L'exemple suivant illustre l'invention sans toutefois en limiter la portée.

### EXEMPLE :

On utilise comme matière de départ un surnageant de cryoprécipité que l'on obtient par décongélation et centrifugation à 0 - 3°C de plasma frais congelé. Ce produit contient du Facteur IX (activité spécifique 0,014 Ul/mg).

### 1. Prépurification

On traite un Lot de surnageant par une résine de DEAE-Sphadex A 50 en présence de sérum physlologique. Après lavage au chlorure de sodium 0,20 M dans un tampon citrate 0,01 M à pH 7,0, on élue au chlorure de sodium 2 M dans un tampon citrate 0,01 M à pH 7,0. On élimine ensuite le sel par ultrafiltration.

L'activité spécifique du Facteur IX dans cette fraction prépurifiée est de l'ordre de 1 Ul/mg.

L'éluat de cette colonne de prépurification contient parmi d'autres protéines, l'albumine, les immunoglobulines, l'antithrombine III l'alpha-antitrypsine et la transferrine,qui peuvent être récupérées, purifiées et concentrées ultérieurement.

### 2. Chromatographie d'échange d'anions

La fraction prépurifiée, désorbée de la résine décrite plus haut et contenant le Facteur IX et les Facteurs II, VII et X, pouvant être additionnée de 1 Ul/ml d'héparine (concentration finale) est injectée sur une colonne de DEAE-Sépharose CL 6B (Pharmacia) équilibrée par un tampon phosphate à pH 6,0.

Pour assurer l'inactivation virale on peut effectuer, avant l'injection sur la colonne, un traitement de type solvant-détergent en utilisant 0,3 % de phosphate de tri-n-butyle et 1 % de Tween 80 (Merck), lequel traitement étant mis en oeuvre à 24 ° C pendant 6 heures.

Les différentes molécules sont désorbées de la colonne par élution avec un tampon de force ionique croissante.
- Récupération de la fraction riche en Facteur VII (proconvertine)
Par élution en tampon 6 mM phosphate et en présence de citrate de sodium 5mM, additionné de chlorure de sodium 0,19 M, on collecte la fraction riche en Facteur VII. On y ajoute de l'antithrombine III à une concentration finale de 1 Ul/ml. Après ultrafiltration pour ajuster la concentration en protéines à environ 30 g/l, on ajuste la concentration d'héparine à 5 Ul/ml et on répartit le concentré à raison de 20 ml par flacon et on lyophilise.
L'analyse du concentré ainsi obtenu révèle que la proconvertine y est présente à une activité de l'ordre de 25 Ul/ml, pour une activité spécifique comprise généralement entre 1,0 et 2,0 Ul/mg de protéine. On y confirme également l'absence des Facteurs de coagulation II, IX et X, qui contribueraient à accroître la thrombogénicité du produit, et celle de la protéine C. Le faible pouvoir thrombogénique du produit est d' ailleurs corroboré par des tests "in vitro" (NAPTT, TGT 50) et "in vivo" (dose efficace 50 (DE₅₀) chez le lapin supérieure à 500 Ul/kg contre environ 30 à 60 Ul/kg pour le PPSB).
Les caractéristiques du produit établissent qu'il apporte un bénéfice significatif dans les traitements anti-hémorragiques des malades présentant un déficit en proconvertine.
- Récupération de la fraction de Protéine C et de Protéine S.
La fraction en question est désorbée de la colonne de DEAE-Sépharose par passage du tampon phosphate en présence de 5 mM de citrate de sodium, à un pH de 6,0, contenant 0,28 M de chlorure de sodium.
L'éluat se caractérise par le contenu suivant :

| | |
|---|---|
| Protéine C | 5 Ul/ml |
| Protéine S | 5 Ul/ml |

Il est dépourvu des Facteurs de coagulation II, VII, IX et X.
- Elution de la fraction contenant les Facteurs II; IX et X.
Cette fraction est éluée par passage du tampon phosphate 5 mM citrate ajusté à un pH de 8,0 et contenant 0,36 M de chlorure de sodium.
Le Facteur IX est présent dans l'éluat en quantité correspondant à une activité spécifique de l'ordre de 5 Ul/mg. Il a été vérifié que cette fraction ne contenait pas de Facteur VII:C.

### 3. Chromatographie d'affinité sur héparine immobilisée et préparation du concentrè de Facteur IX

La dernière fraction éluée de la colonne précédente contient un mélange des Facteurs II, II et X; Elle est ensuite dialysée pour ajuster la force ionique et, après addition de stabilisant à raison de 3 g/l, elle est injectée sur une colonne renfermant un gel chromatographique d'héparine-sépharose équilibré dans le tampon citrate 20 mM, pH 7,4.

On récupère un filtrat contenant le Facteur II.

Le gel est ensuite soumis a une élution par le tampon citrate enrichi en NaCl 0,25 M et on récupère une fraction enrichie en Facteur X.

Par élution plus poussée par le tampon renfermant du chlorure de sodium 0,45 M, auquel on a ajouté 3,5 g/l d'arginine comme stabilisant, on récupère le concentré de Facteur IX.

Dès son élution on y ajoute comme stabilisant pour la lyophilisation 1 g/l de lysine et éventuellement de l'héparine au taux final de 5 Ul/ml. Le produit est alors concentré par ultrafiltration, stérilisé par filtration et lyophilisé.

Les caractéristiques biochimiques moyennes du produit sont indiquées ci-dessous :

| | |
|---|---|
| Protéines (g/l) | 0,19 ± 0,03 |
| Facteur IX:C (Ul/ml) | 25 ± 3 |
| Activité spécifique Facteur IX (Ul/mg) | 130 ± 10 |
| Facteur II:C (Ul:ml) | 0,1 |
| Facteur X:C (Ul/ml) | 0,1 |
| Facteur VII:C (Ul/ml) | 0,1 |
| Facteur IXa (%) | 0 |
| AT III (Ul/ml) | 0 |

Ce concentré de Facteur IX ne contient pas d'autres facteurs de coagulation en quantité décelable (Facteurs XI, XII, prékallikréine, HMW kininogène...) ni de facteurs activés (thrombine, Xa, pka...). Il est également largement dèpourvu d'autres proteines telles que l'alpha-interinhibiteur de trypsine ou le Facteur C₄ qui sont des contaminants majeurs du complexe prothrombinique.

Son activité thrombogénique, telle que déterminée par le modèle de stase sur lapin (test de Wessler) est très faible : DE50 > 450 Ul/kg.

Grâce aux stabilisants le taux de Facteur IX est, après reconstitution, stable au moins huit heures à température ambiante, sans aucune perte d'activité.

Les premières études chez l'homme n'indiquent lors de l'injection, aucune réaction d'intolérance. Les dosages de Facteur V, fibrinogène, plaquettes sanguines n'indiquent aucune variation traduisant une quelconque thrombogénicité du produit.

Les injections sur l'homme indiquent une récupération de Facteur IX de 40 à 45 % et une demi-durée de vie de 20 à 25 heures, soit des valeurs comparables à celles obtenues à l'injection de PPSB.

Ce concentré apparaît comme un produit de grande qualité et d'une sécurité accrue pour le traitement des hémorragies des hémophiles B.

## Revendications

1. Procédé de séparation d'une fraction de plasma humain contenant du Facteur IX, en soumettant la fraction surnageante du cryoprécipité à un procédé chromatographique, ledit procédé étant caractérisé en ce qu'il consiste en les trois étapes chromatographiques suivantes:
a) une prépurification par chromatographie ;
b) une séparation par chromatographie d'échange d'anions sur DEAE-Sepharose et élution sélective avec un tampon dont la force ionique est augmentée par paliers; et
c) une séparation par chromatographie d'affinité sur heparine-sépharose et élution sélective avec un tampon dont la force ionique est augmentée par paliers.

2. Procédé selon la revendication 1, caractérisé en ce que l'étape de prépurification est réalisée par chromatographie sur DEAE-Sephadex.

3. Procédé selon la revendication 1 ou 2, caractérisé en ce que, après l'étape de prépurification, la fraction récupérée présente une activité spécifique du Facteur IX de 0,5 à 1 Ul/mg de protéine.

4. Procédé selon la revendication 3, caractérisé en ce qu'après la séparation du filtrat, la chromatographie est éluée en tampon phosphate-citrate de sodium, additionné de chlorure de sodium pour augmenter progressivement sa force ionique pour séparer trois fractions enrichies respectivement en proconvertine (Facteur VII), en protéine C et protéine S, et en Facteur IX.

5. Procédé selon la revendication 4, caractérisé en ce que le tampon est additionné d'héparine.

6. Procédé selon la revendication 4 ou 5, caractérisé en ce que la concentration en chlorure de sodium du tampon est augmentée à 0,18-0,20 M, à pH légèrement acide, pour éluer la fraction enrichie en proconvertine.

7. Procédé selon la revendication 6, caractérisé en ce que la fraction éluée, enrichie en proconvertine, est additionnée d'héparine et d'antithrombine III.

8. Procédé selon l'une quelconque des revendications 4 à 6, caractérisé en ce que la concentration en chlorure de sodium du tampon est augmentée à 0,26-0,30 M, à pH légèrement acide, pour éluer la fraction enrichie en protéine C et protéine S.

9. Procédé selon l'une quelconque des revendications 4 à 6 et 8, caractérisé en ce que la concentration en chlorure de sodium du tampon est augmentée à 0,34-0,38 M, à pH légèrement basique, pour éluer la fraction enrichie en Facteur IX.

10. Procédé selon l'une quelconque des revendications 1 à 9, caractérisé en ce que la fraction enrichie en Facteur IX est soumise à une chromatographie d'affinité sur héparine-sépharose CL6B.

11. Procédé selon la revendication 10, caractérisé en ce qu'après la séparation du filtrat qui contient le Facteur II, la chromatographie est éluée en tampon citrate additionné de chlorure de sodium pour augmenter progressivement sa force ionique.

12. Procédé selon la revendication 10 ou 11, caractérisé en ce que le tampon d'élution est additionné de 0,5 à 2 g/l de lysine, pour stabiliser le Facteur IX.

13. Procédé selon l'une quelconque des revendications 10 à 12 caractérisé en ce que la concentration en chlorure de sodium du tampon est augmentée à 0,23-0,27 M pour éluer la fraction enrichie en Facteur X.

14. Procédé selon l'une quelconque des revendications 10 à 13, caractérisé en ce que la concentration en chlorure de sodium du tampon est augmentée à 0,43-0,47 M pour éluer la fraction enrichie en Facteur IX.

15. Procédé selon la revendication 14, caractérisé en ce que le tampon d'élution est additionné de 1 à 5 g/l d'arginine pour stabiliser le Facteur IX.

16. Procédé selon la revendication 14 ou 15, caractérisé en ce que la fraction éluée, enrichie en Facteur IX, est additionnée de 0,5 à 2 g/l de lysine, pour stabiliser le Facteur IX en vue de sa lyophilisation.

17. Procédé selon l'une quelconque des revendications 1 à 16, caractérisé en ce qu'il comprend une inactivation virale par traitement au solvant-détergent, après l'étape de prépurification.

## Claims

1. A method of separating a fraction of human plasma containing Factor IX by subjecting the cryoprecipitate supernatant fraction to a chromatographic process, said method being characterised in that it consists of the 3 following chromatographic steps :
a) prepurification by chromatography;
b) separation by anion exchange chromatography on DEAE-Sepharose and selective elution with a buffer whose ionic strength is increased stepwise ;
c) affinity chromatography on heparin/Sepharose and selective elution with a buffer whose ionic strength is increased stepwise ;

2. A method according to claim 1, characterised in that the prepurification step is carried out by chromatography on DEAE/Sephadex.

3. A method according to claim 1 or 2, characterised in that after the prepurification step the specific activity of Factor IX in the fraction recovered is from 0.5 to 1 IU/mg of protein.

4. A method according to claim 3, characterised in that, after separation of the filtrate, the chromatography is carried out using as eluant sodium phosphate-citrate, to which sodium chloride has been added to increase its ionic strength progressively, in order to separate three fractions enriched, respectively, in proconvertin (Factor VII), protein C and protein S, and Factor IX.

5. A method according to claim 4, characterised in that heparin is added to the buffer.

6. A method according to claim 4 or 5, characterised in that the sodium chloride concentration of the buffer is increased to 0.18-0.20M, at a slightly acidic pH, in order to elute the fraction enriched in proconvertin.

7. A method according to claim 6, characterised in that heparin and antithrombin III are added to the eluted fraction that is enriched in proconvertin.

8. A method according to any one of claims 4 to 6, characterised in that the sodium chloride concentration of the buffer is increased to 0.26-0.30M, at a slightly acidic pH, in order to elute the fraction enriched in protein C and protein S.

9. A method according to any one of claims 4 to 6 and 8, characterised in that the sodium chloride concentration of the buffer is increased to 0.34-0.38M, at a slightly alkaline pH, in order to elute the fraction enriched in Factor IX.

10. A method according to any one of claims 1 to 9, characterised in that the fraction enriched in Factor IX is subjected to affinity chromatography on heparin-Sepharose CL6B.

11. A method according to claim 10, characterised in that after separation of the filtrate containing Factor II, the chromatography is carried out using as eluant a citrate buffer to which sodium chloride has been added in order progressively to increase its ionic strength.

12. A method according to claim 10 or 11, characterised in that 0.5 to 2.0 g/l of lysine is added to the elution buffer in order to stabilize Factor IX.

13. A method according to any one of claims 11 to 12, characterised in that the sodium chloride concentration of the buffer is increased to 0.23-0.27M to elute the fraction enriched in Factor X.

14. A method according to any one of claims 10 to 13, characterised in that the sodium chloride concentration of the buffer is increased to 0.43-0.47M to elute the fraction enriched in Factor IX.

15. A process according to claim 14 , characterised in that from 1 to 5 g/l of arginine is added to the elution buffer in order to stabilize Factor IX.

16. A method according to claim 14 or 15, characterised in that from 0.5 to 2 g/l of lysine is added to the eluted fraction that is enriched in Factor IX in order to stabilize Factor IX for the lyophilisation thereof.

17. A method according to any one of claims 1 to 16, characterised in that it comprises a viral inactivation by treatment with solvent-detergent after the prepurification step.

## Patentansprüche

1. Verfahren zur Abtrennung einer Faktor IX enthaltenden menschlichen Plasmafraktion, wobei man die überstehende Fraktion des Kryopräzipitats einem chromatographischen Verfahren unterzieht und dieses Verfahren **dadurch gekennzeichnet** ist, daß es aus den drei folgenden chromatographischen Schritten besteht:
a) einer Vorreinigung durch Chromatographie;
b) einer Trennung durch Anionenaustauschchromatographie an DEA-Sepharose und selektiven Elution mit einem Puffer, dessen Ionenstärke stufenweise erhöht wird;
c) und einer Trennung durch Affinitätschromatographie an Heparin-Sepharose und selektiven Elution mit einem Puffer, dessen Ionenstärke stufenweise erhöht wird.

2. Verfahren gemäß Anspruch 1, dadurch gekennzeichnet, daß der Vorreinigungsschritt durch Chromatographie an DEAE-Sephadex ausgeführt wird.

3. Verfahren gemäß Anspruch 1 oder 2, dadurch gekennzeichnet, daß die nach dem Vorreinigungsschritt gewonnene Fraktion eine spezifische Aktivität an Faktor IX von 0,5 bis 1 IE/mg Protein zeigt.

4. Verfahren gemäß Anspruch 3 , dadurch gekennzeichnet, daß zur Chromatographie nach Abtrennung des Filtrats mit Natriumphosphatcitratpuffer eluiert wird, welchem Natriumchlorid zugesetzt wird, um seine Ionenstärke allmählich zu erhöhen, um drei an Proconvertin (Faktor VII), an Protein c und Protein S beziehungsweise an Faktor IX angereicherte Fraktionen abzutrennen.

5. Verfahren gemäß Anspruch 4, dadurch gekennzeichnet, daß dem Puffer Heparin zugesetzt wird.

6. Verfahren gemäß Anspruch 4 oder 5 , dadurch gekennzeichnet, daß die Konzentration des Puffers an Natriumchlorid bei schwach saurem pH auf 0,18-0,20 M erhöht wird, um die an Proconvertin angereicherte Fraktion zu eluieren.

7. Verfahren gemäß Anspruch 6, dadurch gekennzeichnet, daß der eluierten, an Proconvertin angereicherten Fraktion Heparin und Antithrombin III zugesetzt wird.

8. Verfahren gemäß einem der Ansprüche 4 bis 6, dadurch gekennzeichnet, daß die Konzentration des Pufferz an Natriumchlorid bei schwach saurem pH auf 0,26-0,30 M erhöht wird, um die an Protein C und Protein S angereicherte Fraktion zu eluieren.

9. Verfahren gemäß einem der Ansprüche 4 bis 6 und 8 , dadurch gekennzeichnet, daß die Konzentration des Puffers an Natriumchlorid bei schwach basischem pH auf 0,34-0,38 M erhöht wird, um die an Faktor IX angereicherte Fraktion zu eluieren.

10. Verfahren gemäß einem der Ansprüche 1 bis 9 , dadurch gekennzeichnet, das die an Faktor IX angereicherte Fraktion einer Affinitätschromatographie an Heparin-Sepharose CL6B unterzogen wird.

11. Verfahren gemäß Anspruch 10, dadurch gekennzeichnet, daß nach der Abtrennung des Filtrats, welches den Faktor II enthält, zur Chromatographie mit Citratpuffer eluiert wird, welchem Natriumchlorid zugesetzt wird, um seine Ionenstärke allmählich zu erhöhen.

12. Verfahren gemäß Anspruch 10 oder 11, dadurch gekennzeichnet, das dem Elutionspuffer 0,5 bis 2 g/l Lysin zugesetzt werden, um den Faktor IX zu stabilisieren.

13. Verfahren gemäß einem der Ansprüche 10 bis 12, dadurch gekennzeichnet, das die Konzentration des Puffers an Natriumchlorid auf 0,23-0,27 M erhöht wird, um die an Faktor X angereicherte Fraktion zu eluieren.

14. Verfahren gemäß einem der Ansprüche 10 bis 13, dadurch gekennzeichnet, das die Konzentration des Puffers an Natriumchlorid auf 0,43-0,47 M erhöht wird, um die an Faktor IX angereicherte Fraktion zu eluieren.

15. Verfahren gemäß Anspruch 14, dadurch gekennzeichnet, daß dem Elutionspuffer 1 bis 5 g/l Arginin zugesetzt werden, um den Faktor IX zu stabilisieren.

16. Verfahren gemäß Anspruch 14 oder 15, dadurch gekennzeichnet, daß der an Faktor IX angereicherten Fraktion 0,5 bis 2 g/l Lysin zugesetzt werden, um den Faktor IX hinsichtlich seiner Lyophilisierung zu stabilisieren.

17. Verfahren gemäß einem der Ansprüche 1 bis 16 , dadurch gekennzeichnet, daß es nach dem Vorreinigungsschritt eine virale Inaktivierung durch eine Behandlung mit oberflächenaktiven Waschmittel/Lösungsmittel umfaszt.
